Europäisches Patentamt

(19) European Patent Office

Office européen des brevets

(11) Veröffentlichungsnummer: **0 220 537 B1**

(12) **EUROPÄISCHE PATENTSCHRIFT**

(45) Veröffentlichungstag der Patentschrift: **25.07.90**

(21) Anmeldenummer: **86113681.0**

(22) Anmeldetag: **03.10.86**

(51) Int. Cl.⁵: **A61K 35/26**

(54) Thymusextraktfraktionen enthaltende pharmazeutische Zusammensetzung.

(30) Priorität: **23.10.85 DE 3537707**

(43) Veröffentlichungstag der Anmeldung:
**06.05.87 Patentblatt 87/19**

(45) Bekanntmachung des Hinweises auf die Patenterteilung:
**25.07.90 Patentblatt 90/30**

(84) Benannte Vertragsstaaten:
**AT BE CH DE ES FR GB GR IT LI LU NL SE**

(56) Entgegenhaltungen:
**US-A- 4 444 757**

(73) Patentinhaber: **Dr. Kurt Mulli Nachf. GmbH & Co. KG,
Otto-Hahn-Strasse 2, D-7844 Neuenburg(DE)**

(72) Erfinder: **Jaeger, Karl-Heinz, Dr., Matthofstrand 9,
CH-6005 Luzern(CH)**

(74) Vertreter: **Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86(DE)**

**Beschreibung**

Die Erfindung betrifft eine Thymusextraktfraktionen enthaltende pharmazeutische Zusammensetzung.

Es ist bekannt, daß der Immunapparat des Körpers durch die Thymusdrüse gesteuert wird, und daß solche Funktionen auch von zellfreien Proteinextrakten der Thymusdrüse ausgeübt werden können. In den letzten Jahren wurden deshalb zahlreiche Untersuchungen zur Herstellung, Verwendung und Wirkungsweise von Thymusgewebsextrakten durchgeführt [vgl. z. B. D. Osoba und J. F. A. P. Miller, Nature 199 (1963) 653; A. L. Goldstein und A. White, Contemp. Topics in Immunobiology 1973 339; C. Birr und U. Stollenwerk, Angew. Chemie 91 (1979), 422; Angew. Chemie, Int. Ed. Englisch, 18 (1979) 394]. So wurde z. B. gefunden, daß zellfreie Proteinextrakte der Thymusdrüse von Kälbern, wie das Standardpräparat mit der Bezeichnung Thymosin-Fraktion Nr. 5, in unterschiedlichem Ausmaß Immunmangelerscheinungen unterdrücken, wie eine verminderte Abstoßung von Transplantaten, eine zunehmende Infektionsempfindlichkeit, beschleunigtes Altern und erhöhte Wahrscheinlichkeit des Auftretens von Tumoren. In jüngster Zeit wurde auch berichtet, daß die klinische Anwendung der Thymosin-Fraktion Nr. 5 an Patienten, die an Leukämie oder anderen Krebsarten litten, bereits zu Heilungseffekten geführt hat, besonders bei Lungenkrebs [P. B. Chretien et al., J. D. Cancertreat. Report 62 (1978) 1787 bis 1790].

1977 gelang es A. L. Goldstein et al [J. Proc. Nat 1. Acad. Sci. USA 74 (1977) 725],aus der Thymosin-Polypeptidmischung einen sauren Bestandteil in reiner Form abzutrennen, den sie als Thymosin-$\alpha$ 1 bezeichneten und für den sie auch die Peptidsequenz angaben. Thymosin-$\alpha$ 1 besitzt mit 28 Aminosäuren ein Molekulargewicht von 3107. Da sich Thymosin-$\alpha$1 nur sehr mühsam aus Thymusdrüsen isolieren läßt, wurden auch bereits Methoden zu seiner Totalsynthese vorgeschlagen [Journal of American Chemical Society 101, 1 (1979) 253-254; DE-OS 2 919 592].

Thymosin-$\alpha$ 1 ist mit einem Molekulargewicht von 3107 und 28 Aminosäuren ein relativ großes Polypeptid, dessen Synthese Schwierigkeiten bereitet; es ist zwar auch bekannt, anstelle des Thymosin-$\alpha$ 1 Thymosin-$\alpha$ 1-Fragmente in der Immuntherapie einzusetzen, die sich aufgrund ihres niedrigeren Molekulargewichts einfacher, mit höheren Ausbeuten und mit besserer Reinheit herstellen lassen (vgl. DE-OS 3 100 974), die aber die immunregulierenden oder immunstimulierenden Wirkungen nur in geringerem Ausmaß zeigen.

Es wurde nun gefunden, daß ein Gemisch aus zwei bestimmten Fraktionen eines Thymusgewebe-Extrakts, nämlich aus einer Fraktion aus niedermolekularen Proteinen und/oder Oligopeptiden und einer gelben Fraktion, die Riboflavin, das mit organspezifischen Oligopeptiden assoziiert ist, enthält, hervorragende immunologische Eigenschaften aufweist, die mit denen der Thymosin-Fraktion Nr. 5 oder von Thymosin-$\alpha$ 1 vergleichbar sind und diese in der Wirkungsstärke zum Teil sogar übertrifft. Die erfindungsgemäßen pharmazeutischen Zusammensetzungen sind deshalb hervorragend zur Behandlung von Immunmangelkrankheiten, wie von T-Zellmangelzuständen, des beschleunigten Alterns, der erhöhten Tumorbildungswahrscheinlichkeit und insbesondere von Krebs, geeignet.

Gegenstand der Erfindung ist deshalb eine pharmazeutische Zusammensetzung, die dadurch gekennzeichnet ist, daß sie eine durch Fraktionierung von Thymusextrakt erhältliche Fraktion aus Oligopeptiden und eine gelbe Fraktion, die Riboflavin, das mit organspezifischen Oligopeptiden assoziiert ist, wie durch Wasserextraktion von zerkleinertem Thymus in Gegenwart externer Proteasen, Gewinnung der wäßrigen Phase und Extraktion derselben mit Phenol, Gewinnung der Fraktion aus Oligopeptiden durch Fällung aus der Phenolphase mit Ethanol und der gelben Fraktion durch Chromatographie über Aliminiumoxid und Elution mit Wasser erhältlich, als Wirkstoffe enthält.

Vorzugsweise liegt das Molekulargewicht der Oligopeptide < 2000 Dalton. In der das mit einem organspezifischen Oligopeptid assoziierte Riboflavin enthaltenden Extraktfraktion kann unter "Assoziation" eine Bindung verstanden werden, wie sie z.B. im System Coenzym/prosthetische Gruppe vorliegt.

Ausgangsorgan zur Gewinnung der Extrakte ist Thymus, wobei als Organspender insbesondere Schlachttiere, vorzugsweise Kälber, in Frage kommen.

Überraschenderweise wurde auch gefunden, daß jede einzelne der beiden Extraktfraktionen, also die Fraktionen aus Oligopeptiden oder die gelbe Fraktion, die Riboflavin, das mit organspezifischen Oligopeptiden assoziiert ist, enthält, andere und/oder wesentlich schwächere Wirkungen zeigt als die erfindungsgemäße Zusammensetzung; erst die synergistisch wirkende Kombination der beiden Komponenten zeigt die erfindungsgemäße Wirksamkeit.

Für die erfindungsgemäße Zusammensetzung wurde eine ausgeprägte immunstimulierende Wirksamkeit gefunden, die besser ist als die der bekannten Thymosin-Fraktion Nr. 5. Das Produkt wirkt immunstimulierend und entfaltet hierbei nicht nur eine Wirksamkeit gegenüber Krebszellen, sondern hat sich auch als ausgezeichnet wirksam bei Aids und schweren Formen von Herpes erwiesen. Dies ergibt sich z.B. daraus, daß die T-Suppressorzellen (cytotoxische Subpopulation) im Verhältnis zu den Helferzellen stark erhöht werden.

Der für die Gewinnung der erfindungsgemäßen Fraktionen als Ausgangsprodukt eingesetzte Thymusextrakt kann durch Extraktion von Thymus auf an sich bekannte Weise erhalten werden [vgl. z.B. D. Osoba und J.F.A.P. Miller, Nature 199 (1963) 653; K.H. Jaeger et al., Pharm. Res. Communication 16 (1984) Nr. 6, 559].

Die Herstellung der erfindungsgemäßen Zusammensetzung kann z.B. auf folgende Weise erfolgen: Das aus dem mechanisch zerkleinerten Organ in Gegenwart externer Proteasen (proteolytische Enzyme), wie z.B. von Pankreatinpräparaten oder auch Papain durch Extraktion mit Wasser erhaltene, für eine Zwischenlagerung gegebenenfalls als wäßrige Lösung, Paste oder sprühgetrocknet vorliegende Extrakt wird unter Rühren in Wasser gelöst, die wäßrige Lösung, ggf. nach Abfiltrieren von Trübstoffen, mit Phenol extrahiert; nach Phasentrennung (die wäßrige Phase wird verworfen) wird die Phenolphase mit Äthanol versetzt, wobei die Fraktion aus Oligopeptiden (nachfolgend immer als Peptidfraktion bezeichnet) ausfällt und abfiltriert wird; aus dem Fällungsfiltrat kann die gelbe Fraktion durch Säulenchromatographie isoliert werden. Von den dabei erhaltenen Fraktionen werden die gelb gefärbten Fraktionen, die Riboflavin, das mit organspezifischen Oligopeptiden assoziiert ist, enthalten, (nachfolgend immer als gelbe Fraktion oder gelbe Substanz bezeichnet) abgetrennt und vereinigt; die übrigen Fraktionen werden verworfen. Die so erhaltene gelbe Fraktion kann einer weiteren Auftrennung durch Molekularsiebfraktionierung (Ausschlußchromatographie) unterzogen werden, z.B. an einer präparativen Sephadex® LH-20-Säule mit Wasser (1% Trifluoräthanol) als Elutionsmittel; in diesem Fall werden dann vorzugsweise die bei der zweiten Trennung erhaltenen Hauptfraktionen, die die gelbe Komponente (Riboflavin/Oligopeptide) enthalten, als gelbe Fraktion (gelbe Substanz) der erfindungsgemäßen pharmazeutischen Zusammensetzung verwendet.

Die einzelnen Verfahrensschritte können auf an sich bekannte Weise durchgeführt werden. Die chromatographische Trennung kann unter den für eine Ausschlußchromatographie (Molekularsieb-Effekt) üblichen Bedingungen und mit geeigneten üblichen Füllmitteln (wie z.B. Kieselgel, Sephadex®) und Elutionsmitteln (z.B. Wasser/1% Trifluoräthanol) durchgeführt werden. Die erste Säulenchromatographie des Phenol-Fällungsfiltrates wird vorzugsweise an einer Aluminiumoxidsäule mit Wasser als Elutionsmittel durchgeführt.

Als Wasser zur Lösung des Extraktes und zur Elution wird vorzugsweise ein keimarm filtriertes, keimfreies oder destilliertes Wasser verwendet. Die einzelnen Verfahrensschritte werden zweckmäßigerweise unter Inertgasatmosphäre, wie z.B. unter Stickstoff, durchgeführt, die Filtration vorzugsweise unter den Bedingungen der Sterilfiltration.

Durch Trocknung der Peptidfraktion im Vakuum und schonendes Eindampfen der gelben Fraktion am Rotationsverdampfer im Vakuum oder durch Lyophilisation können die kristallisierten, trockenen Fraktionen erhalten werden; die Trocknung soll dabei in beiden Fällen bei einer Temperatur $\leq 60°C$ durchgeführt werden.

Die Peptidfraktion und die gelbe Fraktion können aber auch als wäßrige Lösung oder Konzentrat, gegebenenfalls nach Zusatz weiterer pharmazeutischer Hilfs- und/oder Trägerstoffe und/oder Wirkstoffe, direkt verwendet werden; bedingt durch die Herstellung kann die Lösung noch geringe Phenolmengen enthalten, die aber nicht störend sind. Insbesondere bei der Herstellung von Konzentraten, aber auch bei Lösungen, kann es auch zweckmäßig sein, Stabilisatoren zuzusetzen, z.B. weiteres Phenol bis zu einer Konzentration von ca. 0,3 bis 1,0 Gew.-%, insbesondere 0,5 Gew.-%, bezogen auf das wäßrige Konzentrat oder die Lösung. Aus der Lösung kann aber, z.B. nach einer Zwischenlagerung, auch die Trockensubstanz, z.B. durch Lyophilisation, isoliert werden, die dann als solche oder zusammen mit weiteren Wirkstoffen und/oder pharmazeutischen Hilfs- und Trägerstoffen appliziert werden kann.

In der Regel entspricht das Mengenverhältnis der Fraktion aus niedermolekularen Proteinen und/oder Oligopeptiden (Peptidfraktion) zu der gelben Fraktion dem Verhältnis, in dem diese beiden Komponenten bei der Organextraktion und nachfolgenden Auftrennung aus dem Extrakt erhalten werden; in einigen Fällen, z.B. für spezielle Anwendungen, kann es aber auch zweckmäßig sein, die eine oder andere Komponente im Überschuß oder in einem anderen als dem sich direkt aus der Extraktion ergebenden Mengenverhältnis anzuwenden.

Als pharmazeutische Hilfs- und Trägerstoffe können alle für einen derartigen Anwendungszweck geeigneten Hilfs- und Trägerstoffe verwendet werden, wobei sich die Auswahl insbesondere nach der vorgesehenen festen oder flüssigen Verwendungsform (Tabletten, Dragees, Kapseln, Sirupe, Lösungen, Injektionslösungen usw.) richtet. Es können auch erfindungsgemäße Extraktgemische mit einem oder mehreren weiteren im Rahmen der Indikation geeigneten Wirkstoffen zur Anwendung kommen.

Die nachfolgenden Beispiele erläutern die Erfindung näher, ohne sie darauf zu beschränken. Wenn nicht anders angegeben, beziehen sich vor- und nachstehend Prozentangaben auf Gewichtsprozent, Temperaturangaben auf Grad Celsius.

Beispiel 1

a) Als Phenol wird chemisch reines Phenol, das den Reinheitskriterien des DAB 7 entspricht, verwendet; als Äthanol absolutiertes und mit Methyläthylketon vergälltes Äthanol.

Ein aus zerkleinertem Rinderthymus erhaltener wäßriger Extrakt wird unter Rühren in Wasser (keimarm filtriert) in einer Konzentration von ca. 20 Gew.-% (bezogen auf Trockenrückstand) gelöst. Die wäßrige Lösung wird mit Phenol (1 Gew.-%) konserviert. Nach einer Zwischenlagerung von 1 bis 10 Tagen wird die wäßrige Lösung durch Filtration geklärt, und die abfiltrierten Trübstoffe werden verworfen.

Das geklärte Filtrat wird mit Phenol versetzt und ca. 5 Minuten gerührt; nach der Phasentrennung (ca. 2 bis 5 Stunden) wird die obere wäßrige Phase (ca. 7 Gew.-% Phenol) abgezogen und die Phenolphase fünfmal mit keimarm filtriertem Wasser gewaschen (jeweils 5 Minuten rühren), danach einmal mit demineralisiertem Wasser. Die sich im Waschwasser lösenden Phenolmengen werden dabei immer wieder ersetzt. Die gewaschene Phenolphase wird unter Rühren in Äthanol eingebracht, wobei die Fällung der Peptide erfolgt. Der nach Filtration über Filterplatten erhaltene Rückstand (Peptidfraktion) wird mit Äthanol gewaschen, bis der Phenolgehalt im Waschalkohol ca.1 Gew.-% ist. Der Filtrationsrückstand wird nach Vortrocknung auf dem Filter (Vakuum, keimarm filtrierte Luft) bei maximal 60°C im Vakuum getrocknet.

b) Physikalisch-chemische Charakterisierung der nach Beispiel 1 a) erhaltenen Peptidfraktion (nachfolgend als 101/83 bezeichnet)

I. UNTERSUCHUNGSANORDNUNGEN UND ERGEBNISSE

1.1 Elementaranalyse:

Die Elemente Kohlenstoff, Wasserstoff und Stickstoff wurden durch Verbrennung im Sauerstoffstrom und anschließende Reduktion der Stickoxide gaschromatographisch als Kohlendioxid, Stickstoff und Wasser bestimmt. Die Schwefelbestimmung erfolgte als Sulfat durch Titration mit Bariumperchlorat.
101/83 : C 46,52% H 6,92% N 14,3% S 0,83%

1.2 Aminosäurenanalyse:

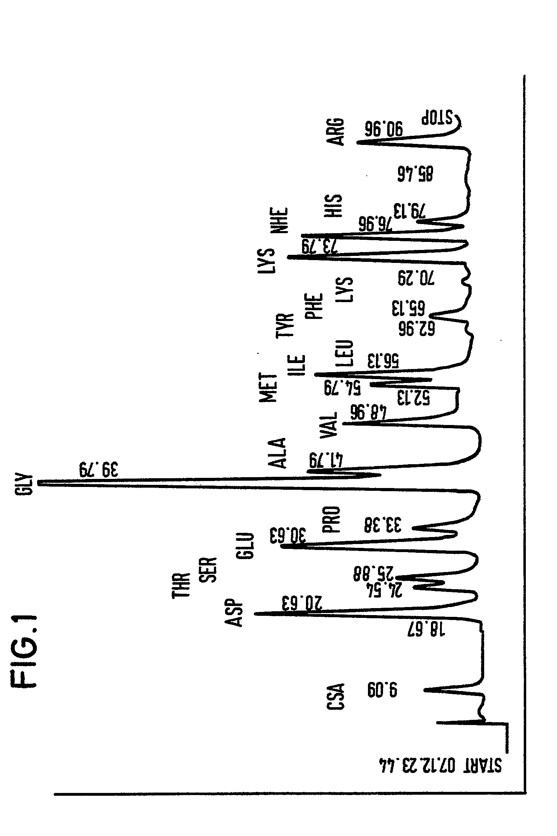

1.2.1 Gehalt an freien Aminosäuren: Eine Substanzprobe 101/83 wurde ohne jede Vorbehandlung quantitativ auf den Gehalt an freien Aminosäuren mithilfe eines automatischen Aminosäuren-Analysators (der Firma Biotronik) nach der Methode von Stein und Moore untersucht. Eine eingewogene Probe wird in einer definierten Menge des Startpuffers des Analysators gelöst und ein Aliquot in das Gerät injiziert.
1 mg der Peptidfraktion 101/83 enthält an freien Aminosäuren (n Mol): 56,0 Cysteinsulfonsäure, 21,6 Asparaginsäure, 5,4 Serin, 6,0 Glutaminsäure, 11,6 Prolin, 17,8 Histidin, ca. 20 Arginin, neben 17,8 nMol nicht eindeutig identifizierbarer Aminosäuren.
1.2.2. Gesamtgehalt an Aminosäuren: Eine eingewogene Menge der Substanz 101/83 wurde in konz. Salzsäure/Wasser (1:1) im Einschlußrohr bei 120°C 24 St. totalhydrolysiert, im Vakuum eingedampft, der Rückstand in einer definierten Menge des Startpuffers (pH 1.8) des Analysators gelöst und ein Aliquot in das Gerät injiziert.
104 µg der Substanz 101/83 (Peptidfraktion aus Thymus) besteht insgesamt aus (n Mol): 13,55 Cysteinsulfonsäure (2,29 µg), 60,95 Asparaginsäure (8,11 µg), 18,1 Threonin (2,16 µg), 22,6 Serin (2,38 µg), 61,1 Glutaminsäure (8,99 µg), 90,3 Prolin (10,4 µg), 148,1 Glycin (11,12 µg), 49,15 Alanin (4,38 µg), 37,0 Valin (4,33 µg), 25,8 Isoleucin (3,38 µg), 36,95 Leucin (4,85 µg), 4,4 Tyrosin (0,797 µg), 17,55 Phenylalanin (2,9 µg), 36,1 Lysin (5,27 µg), 12,65 Histidin (1,96 µg), 34,48 Arginin (6,0 µg).
Demnach entspricht die Peptidfraktion aus Thymus ca. 76% in hydrolysierbarer Form vorliegenden Aminosäuren (peptidischem Material; qualitativ wurden mittels HPLC Nucleotide nachgewiesen). Das Ergebnis zeigt Fig. 1.

2.1 Polyacrylamid-Elektrophorese:

Es wurden Trenngele des Formates $300 \times 150 \times 1$ mm gegossen. Es kamen Gele des Vernetzungsgrades 10, 15 und 20% zur Anwendung. Die Trennungen erfolgten in Gegenwart von Natriumdodecylsulfat (SDS).
Für die Eigenherstellung der Gele benutzte man folgende Stammlösungen:
Unterer Tris-Puffer: 36.34 Tris, 5 ml konz. HCl und 8 ml 10%-ige SDS-Lösung werden mit $H_2O$ auf 200 ml aufgefüllt (pH = 8.8).
Oberer Tris-Puffer: 6.06 g Tris, 4 ml konz. HCl und 4 ml 10%-ige SDS-Lösung werden mit $H_2O$ auf 100 ml aufgefüllt.
Elektrophorese-Puffer: 250 ml Reservoir-Puffer und 10 ml 10%-ige SDS-Lösung werden auf 1000 ml aufgefüllt.
Reservoir-Puffer: 12.0 Tris und 57.6 g Glycin werden mit $H_2O$ auf 1000 ml aufgefüllt.
Acrylamid-Lösung: 30 g Acrylamid und 0.8 g N,N'-Methylen-bis-acrylamid werden mit $H_2O$ auf 100 ml aufgefüllt.
Ammoniumpersulfat: 10%-ige frisch angesetzte Lsg. in $H_2O$
Denaturierungs-Puffer: Elektrophorese-Puffer mit 20% Glycerin, 3% SDS, 3% Mercaptoethanol + Bromphenolblau
Fixierbad: 12.5%-ige TCA
Färbebad: 2.75 g Coomassie-Blau in 500 ml MeOH, 500 ml $H_2O$ und 140 ml AcOH
Entfärbebad: 100 ml MeOH und 150 ml AcOH werden mit $H_2O$ auf 2000 ml aufgefüllt.

|  | Trenngel (10%) | Startgel |
|---|---|---|
| $H_2O$ | 8.33 ml | 3.25 ml |
| Oberer Tris-Puffer: | 5.00 | - |
| Unterer Tris-Puffer: | - | 1.25 |
| Acrylamid-Lösung: | 6.67 | 0.55 |
| Tetramethylethylendiamin (TEMED): | 0.02 | 0.01 |
| Ammoniumsulfat: | 0.01 | 0.02 |

Ausführung:

Konzentrationen der aufgetragenen Proben:
101/83: 7,5 mg /60 µl
Die Proben wurden in je ein 50 µl Denaturierungs-Puffer und 10 µl Bromphenolblau-Lösung aufgenommen. Je 20 µl wurden aufgetragen.
Zur elektrophoretischen Trennung wurden zunächst für 45 Min. bei 13 mA (max. 400 V) die Proben im Sammelgelbereich (Startzone) konzentriert und während 3 Stunden bei konst. 50 mA (8°C Kühlung) elektrophoretisiert.
Zur Entwicklung wurde das Gel anschließend der Sandwichkammer entnommen und 45 Min. bei 22°C im Färbebad behandelt. Überflüssiger Farbstoff wurde im Entfärbebad unter mehrmaligem Wechsel der Flüssigkeit während 8 Std. entfernt.
Ergebnis: Wie für ein Peptidpräparat zu erwarten, läßt sich 101/83 in 10% vernetztem Polyacrylamid-SDS-Gel elektrophoretisch nicht in Einzelkomponenten auftrennen; dies spricht für den Gehalt an niedermolekularen Peptiden.

2.2 Papierelektrophorese:

Bei dem Versuch, die Substanz 101/83 mittels Papierelektrophorese zu untersuchen, wurde festgestellt, daß das Material unter diesen Bedingungen im Hochspannungsfeld nicht auftrennbar ist, sondern eine mit Ninhydrin anfärbbare, für Gemisch-Peptide charakteristisch breit verschmierte Bande bildet.
Puffer: pH 1,9; Zusammensetzung (ml) : Eisessig 15/Ameisensäure 5/Wasser 80 Volumenteile.
Papier: Machery, Nagel und Co., MN-Chromatographiepapier Nr. 214 (23 x 58 cm).
Die Substanzprobe wurde in Wasser /1% Trifluorethanol gelöst an der Startmarkierung aufgetragen.
Die Trennung erfolgte bei 1000V/ 2 St. (0°C).
Entwicklung: Nach Trocknen der Elektrophoresepapiere bei 100°C und Besprühen mit einer Ninhydrin-Lösung wurde das Trennergebnis durch Erhitzen auf 100°C/10 Min. sichtbar gemacht.

3. Endgruppenbestimmung:

Es wurde die Methode der Endgruppenmarkierung mittels Dansylchlorid angewendet, die in der ausgeführten Mikrotecknik in 2-dimensionaler, chromatographischer Auftrennung erlaubt, N-Termini an Aminosäuren, Peptiden und Proteinen im pico-Molbereich ($10^{-12}$ M) nachzuweisen.
Dansylierung: Kleinste Mengen der zu prüfenden Substanz wurden in 0,05 molarem $NaHCO_3$-Puffer mit Dansylchlorid umgesetzt, anschließend sauer hydrolysiert.
Die Hydrolysate werden durch 2-dimensionale Chromatographie auf Mikro-Polyamidplatter untersucht:
1. Dimension, 48% Ameisensäure
2. Dimension, Eisessig/Toluol (1:4,v,v).
Die N-terminal markierten Aminosäuren werden dank ihrer Fluoreszenz im UV-Licht auf den Polyamidplatten identifiziert und photographiert. Die Zuordnung erfolgte nach dem Kartierungsschema der dansylierten Aminosäuren [vgl. H. Laatsch, J.Chromatography 173 (1978) 398-402].

Ergebnis:

Hauptmarkierung: Asp, Glu, Gly, Ala, Pro, Arg, His, NH4, CySO3H, Ser.
Hintergrundmarkierung: Val, Ile, Leu, Tyr, Phe, Lys.

4. Gelchromatographie:

1,455 mg der Peptidfraktion aus Thymus, Probenausgangsnummer 101/83, wurden auf einer Chromatographiesäule (3 x 230 cm), Sephadex® LH 20, mit Wasser (1% Trifluorethanol) als Elutionsmittel, getrennt.
Fraktionierung: 20 ml/Glas/25 Min.
Fließgeschwindigkeit: 0,8 ml/Min
2-Kanal-Detektion: 1. UV (280 nm), 2. Refraktometrie
(Empf. 32 )

Ergebnis:

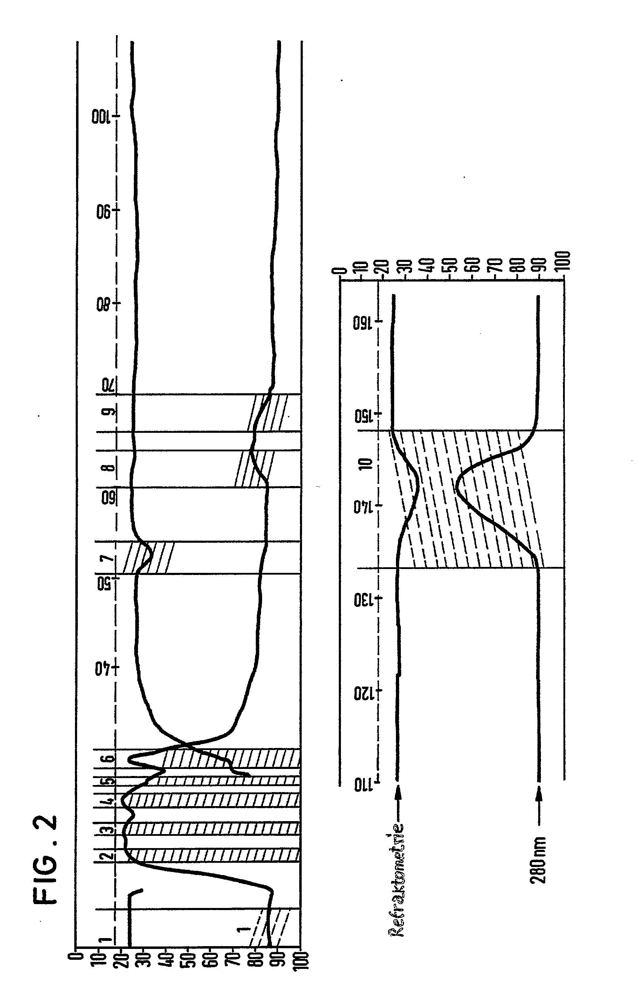

10 Hauptfraktionen: (9-14),(20), (23),(26),(28), (30-31), (50-55), (60-64), (67-70), (134-148);
8 Nebenfraktionen: (15-19), (21-22), (24-25), (27), (29), (32-49), (56-59), (65-66).
Zwischen den Gläsern 70 und 134 verläuft das Chromatogramm entlang der Basislinie; diese Gläser wurden verworfen.
Bei der zusätzlich ausgeführten Dünnschicht-Chromatographie wurden Proben der bei der Gelchromatographie gewonnenen Hauptfraktionen aufgetragen.
DC-Platten: Kieselgel $F_{254}$ (20 x 20 cm; Merck), Schichtdicke 0,250 mm.
Laufmittel: Essigester 15/Pyridin 20/Essigsäure 6/Wasser 11 Volumenteile.
Die gelchromatographische Trennung zeigt die Fig. 2.

## II. AUSWERTUNG DER UNTERSUCHUNGSERGEBNISSE

### 1. Aminosäuren-Analysen:

Vorbemerkung: Bei der Zusammenstellung der quantitativen Daten der Aminosäuren-Analysen vor und nach saurer Totalhydrolyse der zu untersuchenden Substanzen blieben Minimal-Peaks und solche, die bei der automatischen Auswertung vom Analysator nicht zugeordnet werden konnten, unberücksichtigt.
Es ist auffallend, daß freie Aminosäuren nur als minimale Begleitstoffe nachzuweisen sind, wie es für ein Peptid-Präparat zu erwarten ist. Die Identifizierung der freien Aminosäuren ist nicht zweifelsfrei, da bei den Trennläufen die einzelnen Peaks nur als Aufsetzer zu dem Trennmuster der Peptide nachzuweisen sind. Es wird jedoch nachdrücklich darauf hingewiesen, daß besonders bei einer höheren Probenkonzentration (1 mg/500 µl) ein reproduzierbares Peak-Muster erhalten wird, das nicht nur die Identifizierung des Präparates 101/83 als solches, sondern auch dessen Standardisierung leicht ermöglicht: z. B. anhand der Peaks bei 23,73 (Asparaginsäure) und 83,81 (Histidin), sowie den charakteristischen Gipfeln bei 9,56, 57,15 und 71,15, bei denen es sich um charakteristische Peptidauftrennungen handelt.

### 2.1 Polyacrylamid-Elektrophorese:

Anhand des Verhaltens in der Gelelektrophorese läßt sich ableiten, daß 101/83 aus niedermolekularen Peptiden mit einer abgeschätzten Kettenlänge von maximal 15 Aminosäuren/Sequenz besteht. Nur ein Extraktionsprodukt aus Thymus-Gewebe ohne enzymatische Partialhydrolyse läßt sich in dieser Elektrophorese-Technik geringfügig auftrennen.
Wichtig ist jedoch der einfache Befund, daß auch in dieser Technik zu erkennen ist, daß 101/83 an Peptiden reicher ist als die gelbe Fraktion aus Thymus (Beispiel 2).

### 2.2 Papierelektrophorese:

Ein Vergleich des Trennmusters der gelben Fraktion aus Thymus mit dem der Peptidfraktion 101/83 zeigt deutlich, daß im letzten Falle ein komplexes Peptid-Gemisch vorliegt, das sich wegen seiner polyfunktionalen, ionischen Natur nicht ohne chromatographische Vortrennung im elektrischen Hochspannungsfeld aufgliedern und als diskrete Peptidbanden darstellen läßt.

### 3. Endgruppenbestimmung:

Bei der verwendeten Technik werden alle Stickstoff-Funktionen erfaßt, die bei pH 10,5 nicht protoniert sind; es werden also nicht nur Peptid- und Protein-Endgruppen, sondern auch eventuell frei vorhandene Aminosäuren bei dieser Technik markiert.
Da Glycin und Alanin als freie Aminosäuren in dem Präparat nicht enthalten sind, wird deutlich, daß diese beiden die hauptsächlichen Endgruppen der Peptide in 101/83 sind.
Der hohe Gehalt an Asparaginsäure und Cysteinsulfonsäure, sowie Arginin und Histidin in der Analyse freier Aminosäuren findet auch in der Endgruppenbestimmung seine Bestätigung, sodaß diese Aminosäuren nicht aus N-terminaler Peptidbindung stammen können.

Glutaminsäure, Prolin und Serin sind in der Endgruppenbestimmung auffällig vertreten, sodaß diese Aminosäuren auch als Peptidendgruppen angesehen werden können, da sie laut Aminosäuren-Analyse nur in sehr geringer Menge frei in dem Präparat 101/83 vorkommen.

4. Gelchromatographisches Verhalten:

Die Peptidfraktion aus Thymus 101/83 verhält sich bei der Trennung auf Sephadex® LH 20 auffällig als die gelbe Fraktion 69/83 (vgl. Beispiel 2). Die UV-Absorption bei 280 nm der Hauptfraktionen 2 - 6 kann nur auf den Gehalt an Oligonucleotiden zurückgeführt werden, da das Präparat 101/83 seiner Aminosäurenzusammensetzung nach nur insgesamt 6 Gew.-% an UV-absorbierenden Aminosäuren (Tyr, Phe, His) enthält.

Dies wird auch durch den elektrophoretischen Befund auf Zellulose-Dünnschicht und eine qualitative Untersuchung mittels HPLC bestätigt. Wichtig ist, daß durch säulenchromatographische Trennung an Sephadex® LH 20 in Wasser der Oligonucleotid-Gehalt des Präparates von einem Peptidanteil (Fraktion 6) abgetrennt werden kann. Dem Elutionsdiagramm (Fig. 2) ist schließlich zu entnehmen, daß die in den Fraktionen 7 - 10 zusammengefaßten, weit von den Hauptinhaltsstoffen abgetrennten Anteile des Präparates 101/83 nur Substanzen vom Molekülvolumen freier Aminosäuren und Mononucleosiden enthalten können. Dies wird sowohl durch eine zusätzliche elektrophoretische als auch eine dünnschichtchromatographische Untersuchung bestätigt.

Die gelchromatographische Fraktion 6 aus 101/83 ist als Peptid-Leitfraktion des Präparates anzusehen.

Für die erfindungsgemäße pharmazeutische Zusammensetzung kann es zweckmäßig sein, als Peptidfraktion nur die bei der Gelchromatographie (Beispiel 1b, I, 4) erhaltenen Hauptfraktionen oder die Peptid-Leitfraktion (Fraktion 6) einzusetzen.

Beispiel 2

a) Gewinnung der gelben Fraktion:

Das nach Beispiel 1 a) erhaltene Filtrat der Peptidfraktion wird im Vakuum (Schrägrohrschnellumlaufverdampfer) auf ca. 50 % seines Volumens eingedampft (die Waschalkoholfiltrate auf ca. 20 % des Volumens), wobei die Temperatur 60°C nicht übersteigen soll.

Das so erhaltene Konzentratgemisch wird auf Aluminiumoxid-Säulen aufgebracht (Aluminiumoxid Woelm A Super I, Typ W 200 der Fa. Woelm, Eschwege; die Füllung der Säulen erfolgt so, daß die Säule zuerst mit Äthanol gefüllt wird und dann das Aluminiumoxid zugegeben wird; nach einer Standzeit von 12 Stunden ist die Säule einsatzbereit). Nach Aufbringen des Konzentratgemisches auf die Chromatographiesäule wird mit Äthanol nachgewaschen; die Elution erfolgt mit destilliertem Wasser unter Druck (keimarm filtrierte Druckluft). Das Eluat wird nach beendeter Elution im Vakuum auf ca. 20 % des Volumens eingeengt (Schrägrohrverdampfer), und aus dem so erhaltenen Konzentrat dann durch Eindampfen im Rotationsverdampfer ein kristallisiertes trockenes Produkt (gelbe Fraktion) erhalten.

b) Physikalisch-chemische Charakterisierung der nach Beispiel 2 a) erhaltenen Fraktion (nachstehend als 69/83 bezeichnet):

I. VERSUCHSANORDNUNG UND ERGEBNISSE

1.1 Präparative Abtrennung der Flavine durch Ausschluß-Chromatographie.

An einer präparativen Sephadex® LH 20-Säule der Gelbettdimension 3,5 x 240 cm wurden in Wasser (1 % Trifluoräthanol) als Elutionsmittel 5,007g und 16 g der gelben Fraktion (gelbe Substanz) unter Ausnutzung des Molekularsieb-Effektes ausschlußchromatographisch getrennt. Dazu wurden Portionen von je 2 x 5 g und 1 x 6 g in jeweils 5 ml dest. Wasser (1 % Trifluoräthanol) vollständig gelöst und auf das Gelbett aufgetragen. Es wurden Fraktionen von 19 ml/Glas/25 min aufgefangen und der gelchromatographische Trennprozeß kontinuierlich aufgezeichnet 1) durch Messung der UV-Absorption bei 280 nm (Durchfluß-Photometer Uvicord II der Firma LKB) und mittels 2) Durchfluß-Refraktometrie (Waters-Refraktometer, R 4; Dämpfung 64) durch Messung der Dichteveränderung des chromatographischen Eluates.

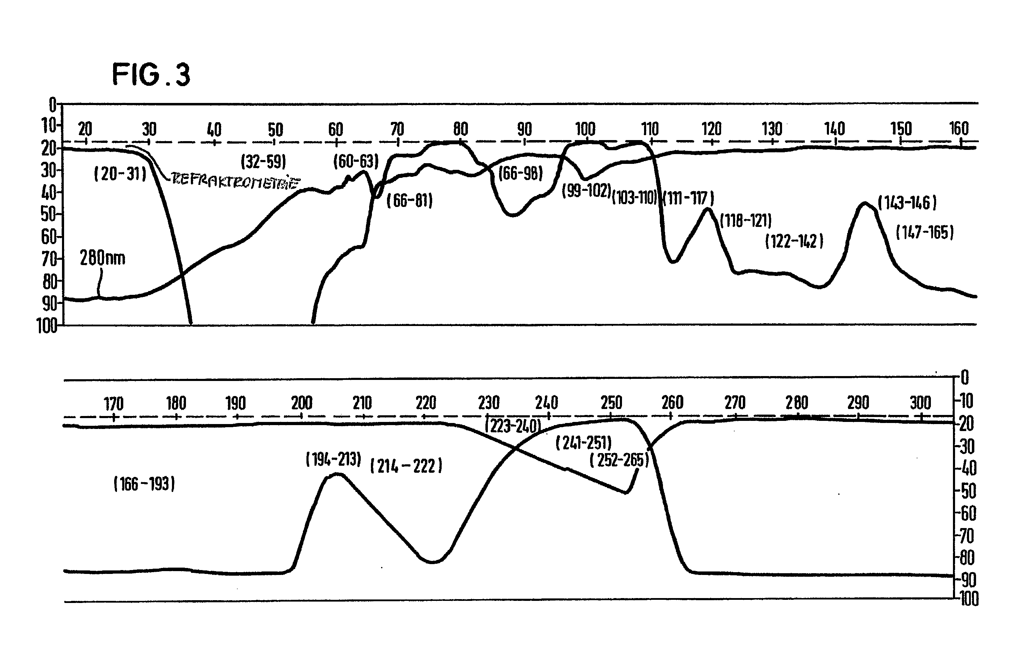

Ein repräsentativer, präparativer Trennlauf ist der Fig. 3 (Elutionsdiagramm) zu entnehmen. In den Trennläufen wanderte eine kräftig gelb gefärbte Komponente als scharf begrenzte Zone durch die Trennsäule und wurde im Bereich der Gläser-Nr. 80-100 aufgefangen, lyophilisiert und ausgewogen.

Ergebnis:

Aus 16 g 69/83 wurden insgesamt 224 mg (1.4 Gew.-%) einer gelben Komponente gewonnen, die nachfolgend analytisch und naturstoffchemisch untersucht wird.

## Elementaranalyse:

|  | C% | H% | N% |
|---|---|---|---|
| Gelbe Kompon. | 52.41 | 6.27 | 14.87 |
| Ribo-flavin | 52.59 | 5.79 | 15.34 |

1.2 Gewinnung lyophilisierter Fraktionen aus der gelchromatographischen Trennung von 69/83.

Orientiert an der Konzentration und UV-Absorption der abgetrennten Komponenten, wurden nach der präparativen Trennung von 5,007 g 69/83 18 Fraktionen zusammengefaßt.

Die Inhalte der entsprechenden Gläser wurden hierzu in vorgewogene Rundkolben entleert, die Gläser dreimal mit dest. Wasser nachgespült, und die Fraktionen entsprechend den vereinigten Gläsern markiert.

Die wässrigen Fraktionen wurden unter Rotation im Vakuum bei -78°C eingefroren und anschließend lyophilisiert (Gefriertrockener Haereus-Leybold, G 2).

Die lyophilisierten Fraktionen wurden in den jeweiligen Rundkolben ausgewogen, luft- und feuchtigkeitsdicht verschlossen (Parafilm) und im Kühlschrank bei + 4°C für weitere Untersuchungen aufbewahrt.

Ergebnis: Aus 5,007 g 69/83 wurden folgende Fraktionen gewonnen:

| Fraktion | Gewicht (mg) | Fraktion | Gewicht (mg) |
|---|---|---|---|
| (20-31) | 38 | (122-142) | 303 |
| (32-59) | 3883 | (143-146) | 4 |
| (60-65) | 316 | (147-165) | 14 |
| (66-81) | 237 | (166-198) | 114 |
| (82-98)* | 94* | (199-208) | 14 |
| (99-102) | 39 | (209-222) | 15 |
| (103-110) | 50 | (223-240) | 7 |
| (111-117) | 16 | (241-251) | 21 |
| (118-121) | 10 | (252-265) | 2 |

* Gelbe Komponente

Die Gesamtmenge der lyophilisierten Fraktionen betrug 5.002 g (99.9%).

1.3a Elementaranalysen dominierender Fraktionen von 69/83

| Fraktion | Gew.-%* | C% | H% | N% | S% |
|---|---|---|---|---|---|
| 20-31 | 0.8 | 34.90 | 5.16 | 39.84 | 0.0 |
| 32-59 | 77.6 | 47.81 | 7.43 | 15.73 | 0.95 |
| 60-65 | 6.3 | 53.16 | 7.38 | 12.17 | 0.70 |
| 66-81 | 4.7 | 52.42 | 7.30 | 15.70 | 0.51 |
| 82-98** | 1.9 | 52.41 | 6.27 | 14.87 | 0.0 |
| 99-102 | 0.8 | 46.95 | 4.68 | 26.58 | 0.0 |
| 103-110 | 1.0 | 50.80 | 5.06 | 16.96 | 0.0 |
| 111-117 | 0.4 | | | | |
| 118-121 | 0.2 | 45.53 | 4.01 | 19.33 | 0.0 |
| 122-142 | 6.1 | | | | |
| 143-146 | 0.1 | 42.89 | 5.09 | 16.55 | 0.0 |
| 147-165 | 0.3 | | | | |
| 166-198 | 2.3 | | | | |
| 199-208 | 0.3 | 36.19 | 7.00 | 26.66 | 0.0 |
| 209-222 | 0.3 | | | | |
| 223-240 | 0.1 | 28.22 | 3.98 | 10.20 | - |
| 241-251 | 0.4 | 25.85 | 3.14 | 7.64 | - |
| 252-265 | <0.1 | - | - | - | - |

\* bezogen auf die Gesamtmenge lyophilisierter Fraktionen (5.002 g)

\*\* gelbe Komponente

1.3b Aminosäure-Analysen dominierender Fraktionen von 69/83

Gehalt an freien Aminosäuren:

Zur Gehaltsbestimmung an freien Aminosäuren wurden 40 µg bis 2 mg eingewogen, ohne jede Vorbehandlung im Startpuffer (pH 1,8) aufgenommen und mit Hilfe eines automatischen Analysators untersucht, um erkennen zu können, welche Endgruppenmarkierungen (s. 1.3 c) auf den Gehalt an mitgeschleppten, peptidassoziierten freien Aminosäuren in den Fraktionen zurückzuführen sind und welcher Anteil der Fraktionen peptidischer Natur ist.

Ergebnis:

1,411 mg der Fraktion (20 - 31) enthalten an freien Aminosäuren (nMol) 52,7 Cysteinsulfonsäure, 15,4 Asparaginsäure, 5,9 Threonin, 7,7 Serin, 5,1 Glycin, 1,8 Lysin und 11,3 $NH_4^{\oplus}$. Aus den ermittelten Daten folgt, daß ca. 1 Gew.-% der Fraktion freie Aminosäuren sind.

195 μg der Fraktion (32 - 59) enthalten an freien Aminosäuren (nMol) 52,1 Cysteinsulfonsäure, 6,6 Asparaginsäure, 20,7 Isoleucin, 25,1 Leucin und 49,9 Phenylalanin. Aus der Gehaltsbestimmung folgt, daß ca. 8 Gew.-% der Fraktion freie Aminosäuren sind.

204 mg der Fraktion (60 - 65) enthalten an freien Aminosäuren (nMol) 2,5 Leucin, 108,2 Phenylalanin, 54,4 Histidin und 27,3 Arginin. Gemäß der Endgruppenbestimmung (s. 1.3 c) konnte der Peak bei einer Retenticnszeit von 73,6 nicht Lysin zugeordnet werden; auch der Gehalt an Histidin und Arginin ist fragwürdig, da die Gestalt der Peaks nicht für diese Aminosäuren charakteristisch ist. Unter diesen Einschränkungen ergibt sich, daß ca. 16 Gew.-% der Fraktion freie Aminosäuren sind.

200 μg der Fraktion (66-81) enthalten an freien Aminosäuren (nMol) 4.0 Thyrosin, 91.9 Phenylalanin, 17 $NH_4^{\oplus}$, 4.2 Histidin und 3.1 Arginin. Daraus ergibt sich, daß ca. 1.9 Gew.-% der Fraktion freie Aminosäuren sind.

40 μg der Fraktion (82-98) enthalten an freien Aminosäuren (nMol) 4.9 Cysteinsulfonsäure und 1.5 Histidin. Daraus folgt, ca. 2.8 Gew.-% der Fraktion sind freie Aminosäuren.

1.995 mg der Fraktion (99-102) enthalten an freien Aminosäuren (nMol) 8.6 Leucin, 1.7 Tyrosin und 10.4 Histidin. Daraus folgt, ca. 0.2 Gew.-% der Fraktion sind freie Aminosäuren.

2.319 mg der Fraktion (103-110) enthalten an freien Aminosäuren (nMol) 1.3 Cysteinsulfonsäure, 1.9 Cystein, 4.5 Leucin, 2.1 Phenylalanin und 5.9 Histidin. Daraus folgt, daß weniger als 0.1 Gew.-% der Fraktion freie Aminosäuren sind.

Gesamtgehalt an Aminosäuren, Bestimmung des peptidischen Anteils der gelchromatographisch gewonnen Fraktionen:

Von den Hauptfraktionen (20-31)-(103-110) wurden Einwaagen von je ca. 40 μg in abgeschmolzenen Ampullen 24 Stdn. bei 120°C in 6 N HCl totalhydrolysiert, im Vakuum zur Trockne eingedampft, im Startpuffer (pH 1.8) aufgenommen und quantitativ mithilfe eines automatischen Analysators auf den Gehalt an Aminosäuren untersucht. Aus den ermittelten Gesamtgehalten an Aminosäuren wurde nach Subtraktion des Gehaltes der Fraktionen an freien Aminosäuren der Peptidgehalt berechnet.

Die Ergebnisse sind in der nachfolgenden Tabelle I zusammengetragen:

EP 0 220 537 B1

Tabelle 1
Gesamt-Aminosäuren- und Peptidgehalt der Hauptfraktionen aus 69/83

| Einwaage [µg] | 40 | 40 | 40 |
| Fraktionen | (20-31) | (32-59) | (60-65) |
| Amino-säuren | G e h a l t [µg] | | |
| Csa | 0.161 | 0.204 | 0.481 |
| Asp | 0.738 | 0.764 | 0.463 |
| Thr | 0.281 | 0.557 | 0.308 |
| Ser | 0.305 | 0.423 | 0.314 |
| Glu | 0.436 | 1.069 | 0.511 |
| Pro | 1.151 | 3.259 | 2.665 |
| Gly | 1.698 | 2.869 | 1.013 |
| Ala | 0.831 | 1.714 | 0.557 |
| Cys | – | – | – |
| Val | 0.379 | 1.125 | 0.851 |
| Met | – | 0.321 | – |
| Ile | 0.247 | 1.177 | 0.914 |
| Leu | 0.234 | 1.842 | 1.623 |
| Tyr | 0.128 | 0.167 | 1.268 |
| Phe | 0.053 | 0.824 | 9.940 |
| Lys | 0.218 | 0.938 | 1.074 |
| $NH_4^{\oplus}$ | 1.307 | 0.152 | 0.121 |
| His | – | 0.439 | 0.635 |
| Arg | – | 1.113 | 0.738 |
| Summe [µg] | 8.167 | 18.957 | 23.476 |
| Gesamt-Gew.-% | 20 | 47 | 59 |
| Gew.-% freier Aminosäuren | – 1 | – 8 | – 16 |
| Peptidgehalt,% | 19 | 39 | 43 |

11

| Einwaage (µg) Frakt. | 40 (66-81) | 50 (82-98)* | 40 (99-102) | 40 (103-110) |
|---|---|---|---|---|
| Amino-säuren | | Gehalt (µg) | | |
| Csa | 0.369 | 0.237 | 0.390 | 0.337 |
| Asp | 0.435 | 0.211 | 0.083 | 0.271 |
| Thr | 0.293 | 0.197 | 0.102 | 0.097 |
| Ser | 0.287 | 0.200 | 0.087 | 0.067 |
| Glu | 0.526 | 0.520 | 0.385 | 0.235 |
| Pro | 1.820 | 2.511 | 0.738 | 0.773 |
| Gly | 1.950 | 0.730 | 5.489 | 1.772 |
| Ala | 0.963 | 0.497 | – | 0.336 |
| Cys | – | – | – | – |
| Val | 0.777 | 0.425 | 0.065 | 0.107 |
| Met | – | 0.396 | 0.184 | – |
| Ile | 0.599 | 0.658 | 0.229 | 0.264 |
| Leu | 1.005 | 1.683 | 0.303 | 0.289 |
| Tyr | 1.905 | 0.306 | 0.351 | 0.825 |
| Phe | 5.765 | 0.601 | 0.445 | 2.935 |
| Lys | 0.213 | 0.141 | 0.095 | 0.133 |
| $NH_4^{\oplus}$ | 0.547 | 0.443 | 1.473 | 1.026 |
| His | 0.123 | – | – | – |
| Arg | 0.281 | 0.137 | 0.413 | 0.331 |
| | 17.850 | 9.893 | 10.932 | 9.798 |
| | 45 | 20 | 27.3 | 25 |
| | – 2 | – 3 | – 0.2 | – 0.0 |
| | 43 | 17* | 27 | 25 |

* = gelbe Komponente

Qualitative Aminosäuren-Analysen der Nebenfraktionen (118-121)-(252-265). Mit der oben geschilderten Methodik wurden Proben von ca. 40 µg nach Totalhydrolyse qualitativ auf den Gehalt an Aminosäuren untersucht, um eine Beziehung zu den Endgruppenbestimmungen (s. 1.3 c) herstellen zu können.

Ergebnis: Die vom Mengenanteil her unbedeutenden, späteren Fraktionen der gelchromatographischen Trennung von 69/83 enthalten nur ca. 10-20 Gew.-% an Peptiden und Aminosäuren, mit abnehmender Tendenz zu späteren Fraktionen. Nach den zugehörigen Elementaranalysen (s. 1.3 a) ist ein Gehalt an Nucleotid-Bruchstücken nicht auszuschließen.

1.3 c Endgruppenbestimmung der Hauptfraktionen

Es wurde die Methode der Endgruppenmarkierung mittels Dansylchlorid angewendet, die in der ausgeführten Mikrotechnik in 2-dimensionaler, chromatographischer Auftrennung erlaubt, N-Termini an Aminosäuren,Peptiden und Proteinen im pico-Mol Bereich ($10^{-12}$ M) nachzuweisen.

Dansylierung: ca. 5 µg der zu prüfenden Substanz wurden in 0.05 Molaren NaHCO₃-Puffer mit Dansylchlorid umgesetzt, anschließend in einer abgeschmolzenen Kapillare 4 Stdn. in 6 N HCl bei 120°C totalhydrolysiert. Die Hydrolysate wurden im Vakuum getrocknet, in Wasser aufgenommen und durch 2-dimensionale Chromatographie auf Mikro-Polyamidplatten untersucht:

1. Dimension, 48% Ameisensäure;
2. Dimension, Eisessig/Toluol (1:4; v,v).

Die N-terminal markierten Aminosäuren wurden dank ihrer Fluoreszenz im UV-Licht auf den Polyamidplatten identifiziert und photographiert. Die Zuordnung erfolgte nach dem Kartierungsschema der dansylierten Aminosäuren (vgl. Beispiel 1 b, I,3).

Ergebnis:

Fraktion (20-31), Hauptmarkierung: His;
Hintergrundmarkierung (in Spuren) : Ser, Asp, Gly.
Fraktion, (32-59), Hauptmarkierung: Gly, His, His*, Arg, Leu;
Hintergrundmarkierung: Asp, Ser, Ala, Val, Pro, Ile, Phe, Tyr, Lys.

Fraktion (60-65), Hauptmarkierung: Phe, Asp, Tyr, Ile, Leu, NH$_4^{\oplus}$, Gly, His, Arg ( ein diffuser Fleck am Rand des linken, unteren Quadranten ist nicht zuzuordnen);
Hintergrundmarkierung: CySO₃H, Ala, Val, Lys (Spuren).

Fraktion (66-81), Hauptmarkierung: Phe, Tyr, NH$_4^{\oplus}$, Gly, Asp;
Hintergrundmarkierung: His, Ser, Thr, Ala, Val, Ile, Leu
Fraktion (82-98), Hauptmarkierung: Tyr;

Hintergrundmarkierung: Phe, Ile, Leu, Val, Ala, NH$_4^{\oplus}$, Gly.
Fraktion (99-102), Hauptmarkierung: His, His*, Tyr;
Hintergrundmarkierung: Glu, Leu, Phe.
Fraktion (103-110), Hauptmarkierung: Gly;
Hintergrundmarkierung: Asp.

2. Analytische und präparative HPLC der unter 1.1 gewonnenen, gelben Komponente zur Reindarstellung einer Flavin-Komponente

Für die nachfolgend geschilderten HPLC-Untersuchungen standen insgesamt 224 mg der gelben Komponente aus 69/83 zur Verfügung, die in wäßriger Lösung stark fluoreszierte. Da sich durch die Elementaranalyse dieser angereicherten Flavin-Komponente der Verdacht auf Riboflavin erhärtet hatte (s. 1), wurde käufliches "Riboflavin für biochemische Zwecke" (Merck) für HPLC-analytische Vergleichszwecke herangezogen.

Trennsäule:

Analytische C₁₈-reversed-phase-Kieselgel-Säulen (4 x 250 mm; Korngröße: 5µ) :

Elutionssystem:

25 % Methanol in Wasser, 10 Min.; weiter 25 Min. ein ansteigender Gradient auf 50 % Methanol, gefolgt von einer Auswaschphase von 12,5 Min. mit reinem Methanol. Danach wird die Säule unter den Anfangsbedingungen äquilibriert.

Analytische HPLC:

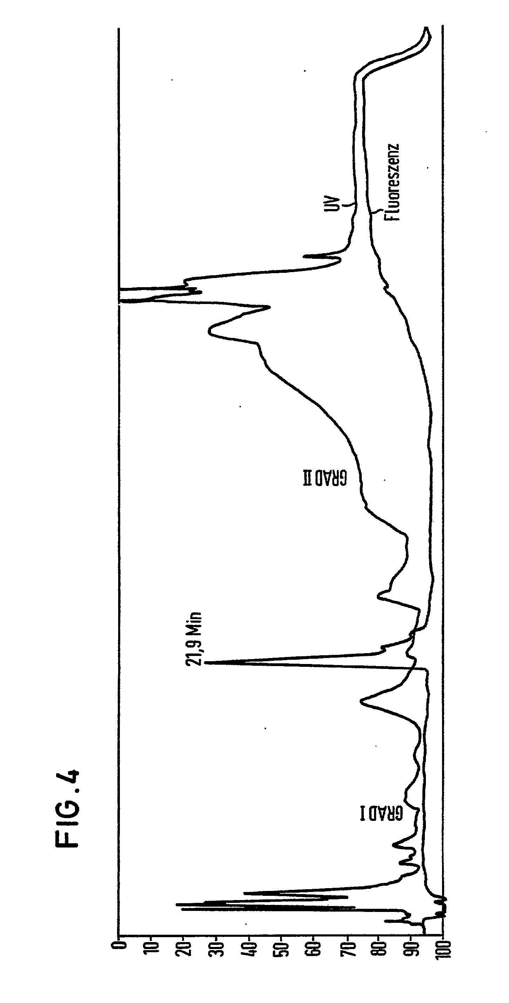

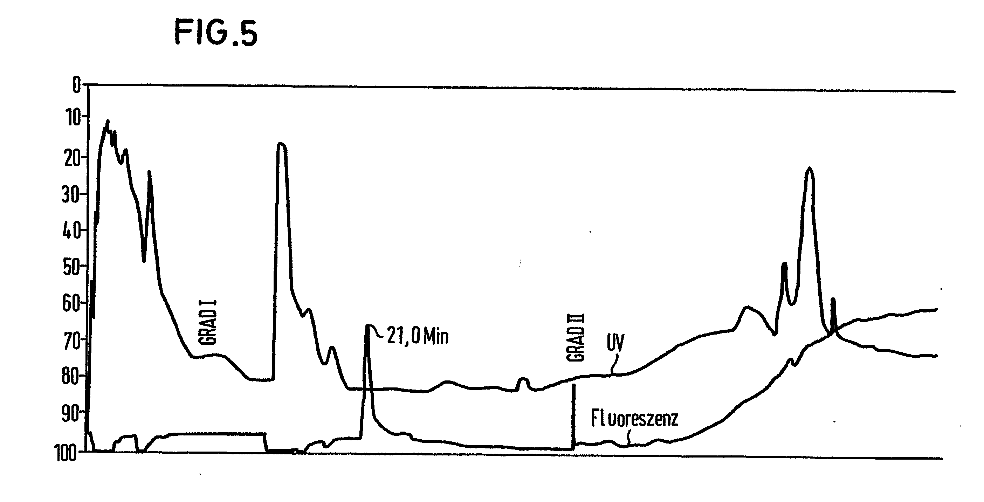

46,25 µg/5 µl Methanol:Wasser 1:1 der gelben Komponente, angereichert nach 1 (c = 9,25 µg/µl) wurde in eine Trennsäule eingespritzt und bei einer Fließrate von 1 ml/min mit dem oben angegebenen Gradientensystem eluiert.
Der Papiervorschub des Schreibers betrug 1 cm/min. Die Detektion erfolgte im UV-Bereich (210 nm) und im Fluoreszenzbereich (excit. 265 nm, emiss. 530 nm).
Das Ergebnis ist in Fig. 4 dargestellt.
In einem technisch identischen Folgelauf wurden an der gleichen analytischen HPLC-Säule 1,205 mg 69/83 (Originalsubstanz) in 5 µl Methanol:Wasser 1:1 gelöst eingespritzt. Das Ergebnis ist in Fig. 5 dargestellt.

In einem weiteren, technisch gleichen Folgeexperiment wurden auf die gleiche analytische Trennsäule 3 µl Riboflavin (MERCK), gelöst in Wasser (c = 714 µg/ml) aufgegeben.

Ergebnis des analytischen Vergleiches:

In der an Sephadex® LH 20 angereicherten, gelben Komponente, sowie in der Originalsubstanz 69/83 ist eine fluoreszierende Substanz enthalten, die exakt mit der gleichen Retentionszeit von 21 Min. aus der analytischen HPLC-Säule austritt wie reines Riboflavin (MERCK).

Eine Konzentrationsberechnung anhand der Fluoreszenzkurve mit Bezug auf die des reinen Riboflavins ergibt für die an Sephadex® LH 20 angereicherte Komponente einen Gehalt von ca. 0.6% an Riboflavin.

Präparative HPLC:

An einer präparativen Säule der Dimension 28 x 250 mm $C_{18}$- reversed-phase-Kieselgel, Korngröße: 5 µ, wurden 129,7 mg der gelben Komponente, angereichert nach 1, gelöst in 4,2 ml Wasser (5 Tropfen 5 N $NH_4OH$) mit einem isokratischen Gemisch aus Methanol/Wasser 18:82 getrennt. Die Fließrate betrug 26 ml/ min bei 130 bar; UV-Detektion bei 254 nm, Papiervorschub des Schreibers 30 cm/St.

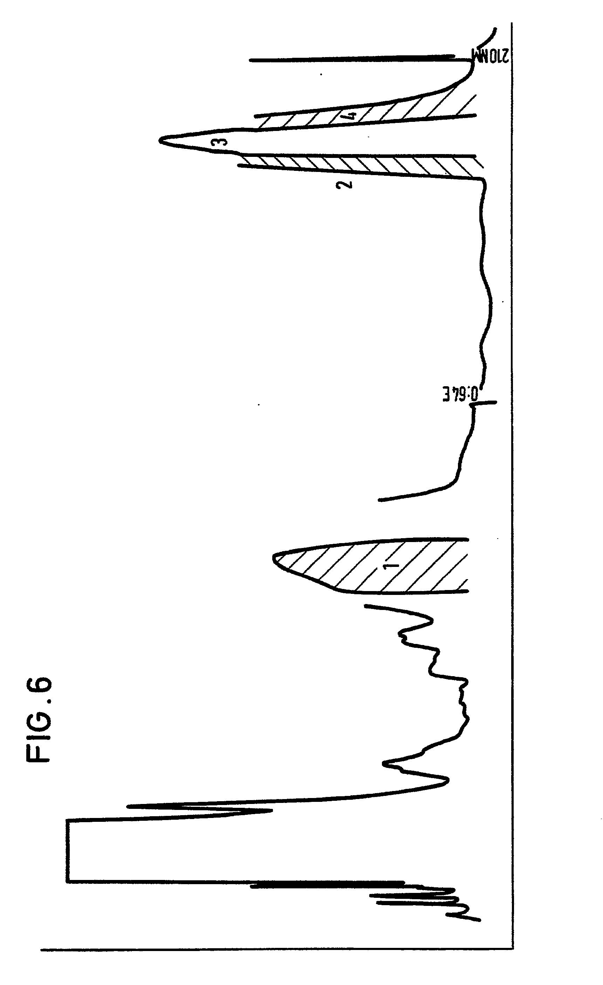

Das Trennergebnis ist in Fig. 6 dargestellt. Es wurden 2 Peaks in vier Fraktionen aufgefangen: Fraktion 1 enthält den ersten, farblosen Peak, der zweite in den Fraktionen 2, 3 und 4 fluoreszierte gelb.

Diese zusammengefaßten Fraktionen ergaben nach Lyophilisation 3,3 mg gereinigter Flavin-Komponente.

Reinheitskontrolle: Die durch präparative HPLC gewonnenen drei Fraktionen der Flavin-Komponente wurden mittels analytischer HPLC an der oben beschriebenen Säule untersucht, wobei folgender, zeitlich gedehnter Gradient verwendet wurde: 10 Min.. 25% Methanol in Wasser, danach in 35 Min. ansteigend auf 60% Methanol in Wasser und anschließend 10 Min. Nachwaschen mit 100% Methanol. Alle übrigen Parameter waren den oben beschriebenen identisch.

Ergebnis:

Alle drei Trennläufe zeigen ein mit reinem Riboflavin identisches Verhältnis der Peakflächen unter der UV- und Fluoreszenz-Kurve. In der absteigenden Schulter der Fluoreszenz-Kurven sind zwei kleine Fluoreszenz-Peaks zu erkennen, die ebenfalls in reinem Riboflavin (Merck) erkannt wurden.

Von der farblosen Fraktion 1 der präparativen HPLC-Trennung wurde eine quantitative Aminosäuren-Analyse nach Totalhydrolyse angefertigt.

55 µg eines mit der Flavin-Komponente vergesellschafteten Peptides enthält folgende Aminosäuren (nMol) : 2.8 Glutaminsäure, 48.9 Prolin, 3.1 Glycin, 1.6 Alanin, 2.1 Valin, 14.4 Isoleucin, 8.0 Tyrosin, 1.2 Phenylalanin, 1.5 Lysin, 14.9 $NH_4^{\oplus}$ und 3.8 Arginin.

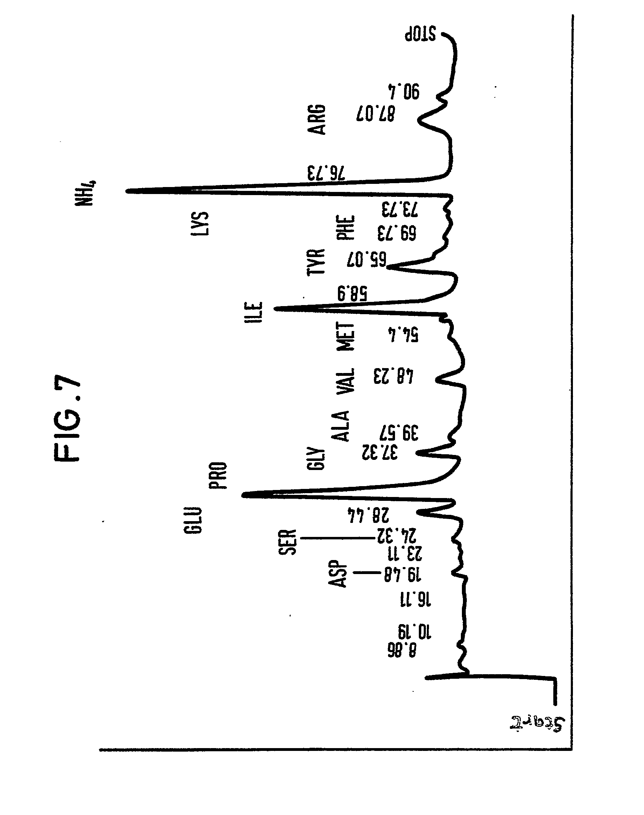

Das Ergebnis ist der Fig. 7 zu entnehmen.

3. Naturstoff-analytische Untersuchungen der aus 69/83 isolierten Flavin-Komponente

Fraktion 3 der präparativen HPLC-Reinigung der aus 69/83 isolierten Flavin-Komponente wurde für naturstoff-analytische Strukturbeweise verwendet.

Kernresonanz-Spektrum: Es wurde ein 360 MHz 1H-NMR-Spektrum der Fraktion 3 aus der präparativen HPLC-Trennung der FlavinKomponente aus 69/83 in $D_2O$ aufgenommen.

Ergebnis:

Das Kernresonanz-Spektrum zeigt alle für Riboflavin charakteristischen Strukturelemente: Die Signale der Methylprotonen am Isoalloxazin-Ring bei 2.46 ppm und bei 2.58 ppm, für den Ribit-Teil bei 3.75 ppm, bei 3.85 - 4.05 ppm und bei 4.35 - 4.46 ppm, sowie für die aromatischen Protonen bei 7.83 - 7.92 ppm.

Für Riboflavin waren in $D_2O$ 14 nicht austauschbare Protonen zu erwarten. Es wurden 14 nicht austauschbare Protonen gefunden.

Massenspektrometrische Identifizierung:

Fraktion 3 der präparativen HPLC-Reinigung der Flavin-Komponente, die mittels Ausschlußchromatographie an Sephadex® LH-20 aus 69/83 angereichert worden war, erbrachte im Vergleich zu Riboflavin (Merck) folgendes Ergebnis:

Reines Riboflavin (Lösungsmittel Wasser) ergibt ein klares Feld-Desorptions-Massenspektrum mit dem Molekülion bei m/z 376 als höchstem Signal und einem (M + Na) +-Ion bei m/z 399. Das zugehörige Feld-Desorptions-Massenspektrum der Flavin-Komponente aus 69/83 zeigt ebenfalls ein starkes Signal für das (M + H) +-Ion 377 und ein noch stärkeres Signal für das Ion (M + Na)+ bei 399 (Lösungsmittel Wasser). Im Vergleich zu authentischem Riboflavin (Merck) enthält das durch präparative HPLC isolierte Riboflavin also erhöhte Mengen an Alkalisalzen.

Das FAB-Massenspektrum (fast atom bombardment) des direkten Vergleiches von Riboflavin (Merck) mit dem aus 69/83 mittels präparativer HPLC isolierten Riboflavin zeigt die Identität der beiden Verbindungen, jedoch in dem aus Thymus gewonnenen Riboflavin ein zusätzliches Signal bei m/z 367 und dessen Glycin-Anlagerungspeak bei m/z 459. Hieraus folgt, daß das mittels präparativer HPLC aus 69/83 isolierte Riboflavin eine Beimengung mit dem Molekulargewicht von 366 enthält. Bei einer höheren Heizleistung von 24 - 26 mA läßt sich der Molekülpeak der unbekannten Substanz bei 367 auch im Feld-Desorptions-Massenspektrum nachweisen.

## II. AUSWERTUNG DER UNTERSUCHUNGSERGEBNISSE

Aus I, 1.3 b ergibt sich, daß die abtrennbare Flavinkomponente 17 Gew.-% an Peptid enthält. Daraus ergibt sich die Frage, ob der Peptidgehalt dieser Komponente chemisch an sie gebunden vorliegt oder aus Gründen lipophiler Wechselwirkung mit dem fluoreszierenden Körper vergesellschaftet auftritt.

Wie durch analytische HPLC an einem Umkehrphasen-Kieselgel ($C_{18}$; 5µ) zweifelsfrei nachgewiesen werden konnte, eluiert die fluoreszierende Substanz mit einer Retentionszeit von 21 Minuten identisch mit authentischem Riboflavin (Merck); dieses Verhalten erwies sich als unabhängig davon, ob die fluoreszierende Substanz in dem Originalpräparat 69/83 direkt mittels analytischer HPLC nachgewiesen wurde, oder aber aus der an Sephadex® LH-20 angereicherten Flavin-Komponente. Zwanglos leitet sich hieraus der Schluß ab, daß eine Peptid-Beimengung nicht an die Flavin-Komponente chemisch gebunden sein kann, da diese sonst unterschiedlich zu authentischem Riboflavin in dem HPLC-System auftreten müßte. Dennoch ist bemerkenswert, daß das mit der Flavin-Komponente vergesellschaftete Peptid in einer sehr stabilen Assoziation zu dieser vorliegen muß, da in der analytischen HPLC in unmittelbarer Nachbarschaft zu dem Fluoreszenz-Peak, vor und nach diesem UV-absorbierende Komponenten eluiert werden (s. hierzu Fig. 4 der Untersuchungen).

Erstaunlich effizient ließ sich der Peptid-Anteil in der Flavin-Komponente, wie er nach der Sephadex® LH-20-Anreicherung vorlag, mittels präparativer HPLC von dem Flavin in einer Basislinien-Trennung separieren. Eine Aminosäure-Analyse des abgetrennten Peptides zeigt durch den Gehalt an Prolin, Isoleucin, Tyrosin und Phenylalanin deutlich den hohen lipophilen Charakter und erklärt damit die enge Wechselwirkung mit dem Flavin.

Beispiel 3:

Herstellung einer pharmazeutischen Zusammensetzung (Injektionslösung)

Zusammensetzung pro 1 ml:
Organextraktfraktionen (Gemisch der zwei nach den Beispielen 1 und 2 erhältlichen Fraktionen) 10,00 mg
Phenol (DAB 7) 4,55 mg
NaCl 5,00 mg
Wasser zur Injektion ad 1,00 ml
Ansatzmenge: 2,2 l (ausreichend für 1000 Ampullen à 2 ml).

Die Inhaltsstoffe werden in 1,5 l Wasser gelöst, danach mit 1 %-iger NaOH ein Ph-Wert von 5,80 eingestellt, und mit Wasser auf 2,2 l aufgefüllt. Die erhaltene Lösung wird sterilfiltriert (0,2 µm Membranfilter), und dann in 2 ml-Ampullen unter Stickstoff-Begasung abgefüllt.

Beispiel 4

Zum Nachweis der immunstimulierenden bzw. immunregulierenden Eigenschaften wurde die so erhaltene erfindungsgemäße Zusammensetzung (Gemisch der nach Beispiel 1a) erhaltenen Peptidfraktion und der nach Beispiel 2a) erhaltenen gelben Fraktion) nach Sterilfiltration und Lyophilisation auf ihre immunbiologische Aktivität an T-Lymphozyten und T-Zellsubpopulationen in heparinisiertem, menschlichem Vollblut gesunder Spender ohne jeden sonstigen Zusatz untersucht. Als Vergleichssubstanz diente sterilfiltrierte und lyophilisierte Thymosin-Fraktion Nr. 5.

a) Gewinnung der Blutproben gesunder Spender:

Für die Blutpräparation standen 11 gesunde Probanden zur Verfügung. Mit Hilfe einer Schmetterlingskanüle wurden je 10 ml Blut aus der Armvene abgenommen, indem das Volumen ohne zu saugen über einen Silikonschlauch von der Kanüle in das heparinisierte Probenglas geleitet wurde. Dieses Vorgehen dient der Schonung der Oberflächen-Marker der T-Lymphozyten, die bei mechanischer Belastung einen Teil der spezifischen Rezeptoren verlieren können.

b) Kulturansätze:

10 ml heparinisiertes Vollblut eines jeden Spenders wurden in vier Portionen zu je 2,5 ml aufgeteilt und in Kulturflaschen mit RPMI 1640-Medium auf je 10 ml aufgefüllt (das RPMI-Medium war mit isotonischer $NaHCO_3$-Lösung auf pH 7,2 gepuffert). Die Kulturflaschen wurden lose verschlossen und ohne jeden sonstigen Zusatz (kein fötales Kälberserum, kein hitzedenaturiertes Albumin) 24 Stunden unter sterilen Bedingungen im $CO_2$-Brutschrank bei 37°C inkubiert.

c) Vorbereitung der Vollblutkulturansätze für die Cytometrie:

Direktmarkierung der T-Lymphocyten und -Subpopulationen im Vollblut mit fluoreszierenden, markerspezifischen Antikörpern (OKT-Seren, Ortho Diagnostic Systems). Aus jedem der Vollblutkulturansätze wurden für fünf Markierungen mit OKT-Seren je 100 µl entnommen und mit je 10 µl der fünf Orthomune ® OKT-Seren, 30 Minuten inkubiert. Anschließend wurden mit dem Lysereagenz (Ortho Diagnostic Systems) auf 2 ml/Probe aufgefüllt, 5 bis 10 Minuten zur Zerstörung der Erythrocyten inkubiert (opake Trübung) und direkt im Cytofluorograph (Ortho) gemessen.

Isolierung der T-Lymphocyten und -Subpopulationen zur Direktmarkierung mit fluoreszierenden, markerspezifischen Antikörpern (OKT-Seren):

Die Vollblutkulturansätze werden zentrifugiert, und das Plasma verworfen. Das Sediment wird in Ficoll-Gradienten 40 Minuten bei 20°C zentrifugiert und der separierte Lymphocytenanteil isoliert. Möglicherweise anhaftende Rest-Erythrocyten werden durch ein Lysereagenz (Ortho Diagnostic Systems) eliminiert, die Lymphocyten mehrmals durch Resuspendieren in PBS und Zentrifugieren gewaschen.

Nach erneuter Resuspension in PBS werden die Lymphocyten auf $10^7$ Zellen/ml eingestellt, hiervon 100 µl Proben mit je 10 µl Orthomune ® OKT-Seren versetzt, 30 Minuten im Eisbad gehalten und dabei nach je 10 Minuten aufgeschüttelt.

Folgende fluoreszierende, monoklonale Antikörper kamen zur Anwendung:

OKT-3, spezifisch für periphere T-Lymphocyten;
OKT-4, spezifisch für T-Helfer-Subpopulationen;
OKT-6, spezifisch für Thymocyten (unreife T-Zellen);
OKT-8, spezifisch für Suppressor-(cytotoxische) T-Subpopulationen;
OKT-11, spezifisch für den E-Rezeptor auf peripheren T-Zellen.

Die Kontroll- und Testkulturen eines jeden Probanden wurden jede für sich in fünf Meßansätzen entsprechend der Markierung mit den spezifischen OKT-Seren aufgegliedert.

Nach Ablauf der Inkubationszeit von 30 Minuten zur Markierung mit den monoclonalen Antikörpern wird durch Zentrifugieren und zweimaliges Waschen mit PBS von überschüssigen OKT-Seren befreit, um unspezifische Fluoreszenz zu vermeiden. Das verbleibende Sediment wird in 1 - 2 ml PBS resuspendiert, so daß im Gesamtvolumen ca. $10^6$ Zellen verbleiben.

d) Durchflußcytometrie mit dem Cytofluorograph (Ortho Diagnostic Systems):

Anhand des Engwinkel-Streulichts, gemessen an einer mit der Meßprobe identischen, unmarkierten Kontrollprobe, wird das Meßfenster am Cytofluorographen auf den für Lymphocyten spezifischen Anteil des Histogramms justiert, wobei ein Autofluoreszenzanteil elektronisch eliminiert wird. Von jeder Meßprobe werden 25 bis 30 000 Zellen/30 Sekunden anhand der grün en Fluoreszenz (spezifisch für die jeweils markierte T-Zellgruppe) und des Engwinkel-Streulichts (spezifisch für alle Lymphocyten) ausgewertet. Die Meßzahlen werden elektronisch gespeichert und als Prozent der Gesamt-Lymphocytenzahl angegeben.

Es wurde gefunden, daß die erfindungsgemäße Zusammensetzung auf die Vermehrung von OKT-8 positiven T-Suppressorzellen (cytotoxische Subpopulation) der Thymosin-Fraktion Nr. 5 in ihrer Wirkung in der Vollblutkultur überlegen ist. Die Peptidfraktion als Teilkomponente des erfindungsgemäßen Präparates zeigt ein dem Präparat verwandtes Wirkungsprofil auf die T-Zell-Subpopulationen, während die andere Komponente des Präparates, die gelbe Fraktion, zwar einen meßbaren Einfluß auf die Vollblutkultur hat, der aber in bezug auf keinen T-Zelltyp als signifikant hervortritt. Damit kommt der Anwesenheit dieser Komponente die Bedeutung eines synergistisch wirkenden Additives zu, das das erfindungsgemäße Präparat in seinem in-vitro-Effekt gegenüber anderen Thymus-Präparaten wie z. B. Thymosin-Fraktion Nr. 5, deutlich verstärkt.

Im in-vitro-System (Vollblutkultur) zeigte sich ein deutlicher Effekt auf die Ausprägung von Oberflächenmerkmalen, die für Subpopulationen von T-Lymphozyten typisch sind und durchflußzytometrisch mit monoklonalen Antikörpern nachgewiesen wurden. Insbesondere zeigte sich eine Zunahme der Merkmale, die für "Suppressor"- bzw. "zytotoxische" T-Zellen charakteristisch sind (OKT 8-positive Zellen). Dieser Effekt war bei einigen Individuen (als "Responder" bezeichnet), aber auch in der Statistik, größer als bei der Referenzsubstanz "Thymosin-Fraktion Nr. 5".

Aus Untersuchungen ergibt sich eine klinische Anwendbarkeit des erfindungsgemäßen Präparates bei Patienten mit anomalem Bestand an Subpopulationen, insbesondere an "Suppressor"-/zytotoxischen (OKT 8-positiven) Zellen. Ein solcher Befund ist vor allem bekannt für einige als Autoimmunkrankheiten beschriebene Bindegewebserkrankungen, bei denen fast ausnahmslos eine Verminderung der OKT 8-positiven Subpopulation besteht (z. B. rheumatoide Arthritis, Lupus erythematodes, Sklerodermie und Sjögren-Syndrom). In diesen Fällen ist auch im "in-vitro"-System ein noch ausgeprägterer Effekt auf die Erhöhung der Zahl der OKT 8-positiven Zellen zu erwarten; dabei dürfte auch die Wirkstoffdosis geringer gehalten werden können. Bei der Applikation des erfindungsgemäßen Präparates an Patienten des beschriebenen rheumatischen Formenkreises muß vor und während der Anwendung der zelluläre Immunstatus an peripheren Lymphozyten mit OKT-Seren überprüft werden.

Die Art und Menge der Verabreichung des erfindungsgemäßen Präparates richtet sich dabei insbesondere nach der Art und Schwere der Erkrankung und dem Allgemeinbefinden und der Sensibilität des Patienten.

## Patentansprüche für die Vertragsstaaten: BE, CH, DE, ES, FR, GB, IT, LI, LU, NL, SE, GR

1. Pharmazeutische Zusammensetzung, dadurch gekennzeichnet, daß sie eine durch Fraktionierung von Thymusextrakt erhältliche Fraktion aus Oligopeptiden und eine gelbe Fraktion, die Riboflavin, das mit organspezifischen Oligopeptiden assoziiert ist, enthält, wobei die beiden Fraktionen durch Wasserextraktion von zerkleinertem Thymus in Gegenwart externer Proteasen, Gewinnung der wäßrigen Phase und Extraktion derselben mit Phenol, Gewinnung der Fraktion aus Oligopeptiden durch Fällung aus der Phenolphase mit Ethanol und der gelben Fraktion durch Chromatographie über Aluminiumoxid und Elution mit Wasser erhältlich sind, als Wirkstoffe enthält.

2. Pharmazeutische Zusammensetzung nach Anspruch 1, dadurch gekennzeichnet, daß das mittlere Molekulargewicht der Oligopeptide <2000 Dalton ist.

3. Pharmazeutische Zusammensetzung nach Anspruch 1 oder 2, dadurch gekennzeichnet, daß die Fraktion aus Oligopeptiden die Aminosäuren Cysteinsulfonsäure, Asparaginsäure, Threonin, Serin, Glutaminsäure, Prolin, Glycin, Alanin, Valin, Isoleucin, Leucin, Thyrosin, Phenylalanin, Lysin, Histidin und Arginin in freier oder gebundener Form enthält und mindestens 75 Gew.-% der Aminosäuren in hydrolysierbarer Form vorliegen.

## Patentansprüche für den Vertragsstaat: AT

1. Verfahren zur Herstellung einer pharmazeutischen Zusammensetzung, dadurch gekennzeichnet, daß zerkleinerter Thymus in Gegenwart externer Proteasen mit Wasser extrahiert, die wäßrige Phase gewonnen und mit Phenol extrahiert wird, aus der Phenolphase eine Fraktion aus Oligopeptiden durch Fällung mit Ethanol und eine gelbe Fraktion, die Riboflavin, das mit organospezifischen Oligopeptiden assoziiert ist enthält, durch Chromatographie über Aluminiumoxid und Elution mit Wasser erhalten wird und die beiden Fraktionen vereinigt werden.

2. Verfahren zur Herstellung einer pharmazeutischen Zusammensetzung nach Anspruch 1, dadurch gekennzeichnet, daß das mittlere Molekulargewicht der Oligopeptide <2000 Dalton ist.

3. Verfahren zur Herstellung einer pharmazeutischen Zusammensetzung nach Anspruch 1 oder 2, dadurch gekennzeichnet, daß die Fraktion aus Oligopeptiden die Aminosäuren Cysteinsulfonsäure, Asparaginsäure, Threonin, Serin, Glutaminsäure, Prolin, Glycin, Alanin, Valin, Isoleucin, Leucin, Thyrosin, Phenylalanin, Lysin, Histidin und Arginin in freier oder gebundener Form enthält und mindestens 75 Gew.-% der Aminosäuren in hydrolysierbarer Form vorliegen.

## Claims for the Contracting States: BE, CH, DE, ES, FR, GB, IT, LI, LU, NL, SE, GR

1. Pharmaceutical composition, characterised in that it contains, as active material, a fraction obtainable by fractionation of thymus extract of oligopeptides and a yellow fraction, which contains riboflavin which is associated with organ-specific oligopeptides, whereby the two fractions are obtainable by water extraction of comminuted thymus in the presence of external proteases, obtaining of the aqueous phase and extraction thereof with phenol, obtaining of the fraction from oligopeptides by precipitation from the phenol phase with ethanol and the yellow fraction by chromatography over aluminium oxide and elution with water.

2. Pharmaceutical composition according to claim 1, characterised in that the average molecular weight of the oligopeptides is <2000 Dalton.

3. Pharmaceutical composition according to claim 1 or 2, characterised in that the fraction of oligopeptides contains the amino acids cysteine-sulphonic acid, aspartic acid, threonine, serine, glutamic acid, proline, glycine, alanine, valine, isoleucine, leucine, thyrosine, phenylalanine, lysine, histidine and arginine in free or bounded form and at least 75 wt.% of the amino acids are present in hydrolysable form.

## Claims for the Contracting State: AT

1. Process for the preparation of a pharmaceutical composition, characterised in that comminuted thymus is extracted with water in the presence of external proteases, the aqueous phase is recovered and extracted with phenol, from the phenol phase is obtained a fraction of oligopeptides by precipitation with ethanol and a yellow fraction, which contains riboflavin, which is associated with organospecific oligopeptides, by chromatography over aluminium oxide and elution with water and the two fractions are combined.

2. Process for the preparation of a pharmaceutical composition according to claim 1, characterised in that the average molecular weight of the oligopeptides is <2000 Dalton.

3. Process for the preparation of a pharmaceutical composition according to claim 1 or 2, characterised in that the fraction of oligopeptides contains the amino acids cysteine sulphonic acid, aspartic acid, threonine, serine, glutamic acid, proline, glycine, alanine, valine, isoleucine, leucine, thyrosine, phenylalanine, lysine, histidine and arginine in free or bound form and at least 75 wt.% of the amino acids are present in hydrolysable form.

**Revendications pour les Etats contractants: BE, CH, DE, ES, FR, GB, IT, LI, LU, NL, SE, GR**

1. Composition pharmaceutique, caractérisée en ce qu'elle contient comme constituants actifs une fraction d'oligopeptides, qui peut être obtenue par fractionnement d'un extrait de thymus, et une fraction jaune, qui contient de la riboflavine qui est associée à des oligopeptides organospécifiques, les deux fractions pouvant être obtenues par extraction à l'eau du thymus réduit en fragments en présence de protéases externes, isolement de la phase aqueuse et extraction de celle-ci au phénol, isolement de la fraction d'oligopeptides par précipitation à l'éthanol à partir de la phase phénolique, et de la fraction jaune par chromatographie sur oxyde d'aluminium et élution à l'eau.

2. Composition pharmaceutique selon la revendication 1, caractérisée en ce que le poids moléculaire moyen des oligopeptides est inférieur à 2000 Daltons.

3. Composition pharmaceutique selon la revendication 1 ou 2, caractérisée en ce que la fraction d'oligopeptides contient les acides aminés acide cystéinesulfonique, acide aspartique, thréonine, sérine, acide glutamique, proline, glycine, alanine, valine, isoleucine, leucine, thyrosine, phénylalanine, lysine, histidine et arginine sous forme libre ou liée, et en ce qu'au moins 75% en poids des acides aminés sont sous forme hydrolysable.

**Revendications pour l'Etat contractant: AT**

1. Procédé de préparation d'une composition pharmaceutique, caractérisé en ce que l'on extrait à l'eau du thymus réduit en fragments en présence de protéases externes, l'on isole la phase aqueuse et on l'extrait au phénol, l'on isole à partir de la phase phénolique une fraction d'oligopeptides par précipitation à l'éthanol et une fraction jaune qui contient de la riboflavine qui est associée à des oligopeptides organospécifiques, par chromatographie sur oxyde d'aluminium et élution à l'eau, et l'on réunit les deux fractions.

2. Procédé de préparation d'une composition pharmaceutique selon la revendication 1, caractérisé en ce que le poids moléculaire moyen des oligopeptides est inférieur à 2000 Daltons.

3. Procédé de préparation d'une composition pharmaceutique selon la revendication 1 ou 2, caractérisé en ce que la fraction d'oligopeptides contient les acides aminés acide cystéine-sulfonique, acide aspartique, thréonine, sérine, acide glutamique, proline, glycine, alanine, valine, isoleucine, leucine, thyrosine, phénylalanine, lysine, histidine et arginine sous forme libre ou liée, et en ce qu'au moins 75% en poids des acides aminés sont sous forme hydrolysable.

FIG.1

EP 0 220 537 B1

FIG . 2

Refraktometrie →

280 nm →

EP 0 220 537 B1

FIG.3

## FIG.4

21,9 Min

GRAD I

GRAD II

UV

Fluoreszenz

EP 0 220 537 B1

# FIG.5

EP 0 220 537 B1

FIG.6

EP 0 220 537 B1

# FIG.7